# EUROPEAN PATENT APPLICATION

(11) **EP 1 634 593 A2**
(43) Date of publication of application: **15.03.2006**
(21) Application number: 05026817.6
(22) Date of filing: 13.11.2000
(51) Int. Cl.: A61K 31/315, A61K 31/555, A61K 31/381, A61P 3/10, C07F 3/06

(54) **Hypoglycemics comprising organic zinc (II) complexes**

(30) Priority: 30.11.1999 JP 34005899; 18.05.2000 JP 2000145849
(62) Divisional of application: 00974960.7
(71) Applicant: Japan Science and Technology Agency, Kawaguchi-shi Saitama (JP)
(72) Inventor: Kojima, Yoshitane, Izumi-shi Osaka 594-0013 (JP); Sakurai, Hiromu, Nagaokakyo-shi Kyoto 617-0825 (JP)
(74) Representative: Cresswell, Thomas Anthony

(57) **Abstract**

Hypoglycemics having a lower toxicity than vanadium and an adequate stability and exerting an adequate fat-soluble insulin-like effect; and medicinal preparations having an insulin-like effect and being useful as preventives and remedies for diabetes. Hypoglycemics comprising organic zinc (II) complexes which contain as the ligand selected from among ligands having pyridine ring, ligands having pyrone ring, ligands having thiazolidine ring, ligands comprising amino acids, ligands comprising 2-aminothio-phenols and ligands comprising pyrrolinedithiocarbamic acids; and medicinal preparations containing the above-described organic zinc (II) complexes which are useful as remedies for diabetes.

## Description

### Technical Field

The present invention relates to a hypoglycemic agent comprising organic zinc (II) complexes. More particularly, the invention relates to a hypoglycemic agent comprising an organic zinc (II) complex having as a ligand a pyridine ring-containing ligand, a pyrone ring-containing ligand, a thiazolidine ring-containing ligand, a thiophene ring-containing ligand, a ligand comprising an amino acid, a ligand comprising a 2-aminothiophenol or a ligand comprising a dithiocarbamic acid. The invention also relates to an oral formulation comprising organic zinc (II) complexes which have a hypoglycemic effect and an insulin-like effect.

### Background of the Invention

In spite of a rapid advance of medical technologies, various adult diseases such as Alzheimer's disease, ischemic heart failure and diabetes are still remaining as substantial problems in Japan. Among these diseases, a diabetes is prevailing in a large number of latent patients, which is still increasing continuously year by year. Currently, the diabetes is defined as a disease which exhibits an abnormal glucose, protein or lipid metabolism due to an absolute or relative deficiency of insulin and which leads to a characteristic complication of the diabetes, such as a nephropathy, retinopathy, neuropathy and the like, resulting from a chronic hyperglycemia. The diabetes is classified by World Health Organization (WHO) broadly into two types, namely, type I (insulin-dependent) diabetes and type II (insulin-non-dependent) diabetes. In the type I diabetes due to an absolute deficiency of insulin, B cells in pancreas are destroyed due to an autoimmune islet inflammation, resulting in suppressed synthesis or secretion of the insulin. Accordingly, an insulin injection is the only therapy which is possible practically. Such insulin is painful for patients, and thus therapeutic agents as substitutes for the insulin are expected all over the world.

On the other hand, a type II diabetes involves a relative low sensitivity of an insulin to cells. Factors of the onset of the type II diabetes are thought due to obesity, stress or lack of exercise.

The reduced effect of the insulin in the diabetes leads to glucose consumption impairment in tissues inducing an energy production, which is compensated by a promoted neutral fat decomposition in fat cells, and resulting in a promoted release of free fatty acids (FFA) as an energy source. While the neutral fat decomposition is promoted by an activation of a hormone-sensitive lipase, this enzyme is activated by catecholamine, glucagon, adrenotropic hormone and the like but inactivated by insulin. In diabetes, the release of FFA from fat tissues is promoted not only by an insulin deficiency but also by an increased glucagon secretion.

In addition, the type I diabetes can currently be treated only by a subcutaneous injection of an insulin, and oral therapeutic agents as substitutes for the insulin are desired to be developed. One of such therapeutic agents is vanadyl sulfate which has already been employed in clinical trials in the United States. On the other hand, zinc (II) ion known to be less toxic when compared with vanadium had become to be known to have an insulin-like activity since about 1980 (L.Coulston and P.Dandona, "Insulin-like effect of zinc on adipocytes", Diabetes,29, 665-7(1980); J.M.May and C.S.Contoreggi, "The mechanism of the insulin-like effects of ionic zinc", J.Biol.Chem., 257, 4362-8(1982); A.Shisheva, D.Gefel and Y.Shechter, "Insulin-like effect of zinc ion in vitro and in vivo" 'Zn²⁺ is the first agent other than vanadate that on oral administration is able to restore tissue ability to metabolism glucose), Diabetes, 41, 982-8 (1992)).

Since vanadyl sulfate and zinc (II) ion (zinc sulfate or zinc chloride) are inorganic salts, they have difficulties in penetrating biological membranes and being incorporated into tissues. For the purpose of overcoming such difficulties, a hypoglycemic agent, which is less toxic than vanadium, which exhibited appropriate degrees of a stability and a fat-soluble insulin-like effect, and which is more effective than vanadyl complexes, is desired to be developed.

### Disclosure of the Invention

The present invention provides hypoglycemic agents which are less toxic when compared with vanadium, which exhibit appropriate degrees of stabilities and fat-soluble insulin-like effects. The invention also provides a hypoglycemic agent capable of being administered orally and having an insulin-like effect.

We made an extensive study on zinc (II) compounds, and f inally discovered that organic zinc (II) complexes, especially such organic zinc (II) complexes having as a ligand of a pyridine ring-, pyrone ring-, thiazolidine ring- or thiophene ring-containing compound as well as an amino acid, 2-aminothiophenol or dithiocarbamic acid are less toxic when compared with those of corresponding vanadium complexes and exhibit appropriate degrees of stabilities and fat-soluble insulin-like effects, and that such organic zinc (II) complexes can be administered orally.

Accordingly, the invention relates to hypoglycemic agents which comprise organic zinc (II) complexes, which have hypoglycemic effect and insulin-like effect and thus they are useful as a prophylactic or therapeutic agent against the diabetes.

More particularly, the invention relates to hypoglycemic agents comprising organic zinc (II) complexes having as a ligand of a pyridine ring-containing ligand, a pyrone ring-containing ligand, a thiazolidine ring-containing ligand, a thiophene ring-containing ligand, a ligand comprising an amino acid, a ligand comprising a 2-aminothiopgenol or a ligand comprising a dithiocarbamic acid.

The invention also relates to an oral formulation comprising organic zinc (II) complexes. Moreover, the invention relates to an oral formulation comprising hypoglycemic agents which have hypoglycemic effect and insulin-like effect and thus they are useful as a prophylactic or therapeutic agent against the diabetes.

Furthermore, the invention relates to a pharmaceutical composition comprising organic zinc (II) complexes together with a pharmaceutically acceptable carrier.

Moreover, the invention relates to a use of organic zinc (II) complexes for producing a hypoglycemic agent useful for a prophylactic or therapeutic agent against the diabetes. The invention relates to a method for preventing or treating a hyperglycemic disease such as the diabetes which comprises administering a therapeutically effective amount of organic zinc (II) complexes to patients.

While an inorganic zinc (II) ion compound has already been known to have an insulin-like effect, its unsatisfactory efficacy and difficulty in penetrating a biological membrane to be incorporated into an in vivo tissue makes it difficult to exhibit any in vivo effect.

We made an extensive study on a zinc (II) which can penetrate a biological membrane and can readily be incorporated into an in vivo tissue, and finally discovered that organic zinc (II) complexes have each sterically bulky organic moiety and zinc (II) as its center metal which form structures allowing them to penetrate biological membranes and to be incorporated readily into in vivo tissues and also have sufficient insulin-like effect and hypoglycemic effect.

### Brief Description of the Drawings

Figure 1 shows the effect of an inventive organic zinc (II) complex on the release of a fatty acid from fat cells. In Figure 1, 1 denotes a blank, 2 denotes a control, 3 to 5 denote positive controls and 6 to 26 denotes inventive compounds.
Figure 2 shows the effect of an inventive organic zinc (II) complex on the release of a fatty acid from fat cells. In Figure 2, 1 denotes a blank, 2 denotes a control, 3 to 5 denote positive controls and 6 to 14 denotes inventive compounds.
Figure 3 shows the blood glucose curves observed in a glucose tolerance test in KK-A^{y} mice treated with or without an inventive organic zinc (II) complex.
Figure 4 shows the blood glucose curves observed in a glucose tolerance test in humans (diabetes patients and normal healthy volunteers).
Figure 5 shows a full-range chart of the infrared absorption spectrum (IR) of the inventive bis (3-hydroxy-2-methyl-4-pyronate) zinc (II) complex Zn(maltol)₂/2.6H₂O.
Figure 6 shows the change in the blood glucose after a treatment with the inventive organic zinc (II) complex Zn(PA)₂ for 15 days.
Figure 7 shows the change in the blood glucose after a treatment with the inventive organic zinc (II) complex Zn(maltol)₂ for 15 days.
Figure 8 shows the change in the blood glucose after a treatment with the inventive organic zinc (II) complex Zn(Cys-OMe)₂ for 13 days.
Figure 9 shows the change in the body weight after a treatment with the inventive organic zinc (II) complex Zn(PA)₂ for 15 days.
Figure 10 shows the change in the body weight after a treatment with the inventive organic zinc (II) complex Zn(maltol)₂ for 15 days.
Figure 11 shows the change in the body weight after a treatment with the inventive organic zinc (II) complex Zn(Cys-OMe)₂ for 13 days.
Figure 12 shows the results of a glucose tolerance test after a treatment with an inventive organic zinc (II) complex for 15 days.

### Best Mode for Carrying Out the Invention

Organic zinc (II) complexes according to the invention are preferably zinc (II) complexes of Zn(N₂O₂), Zn(O₄), Zn(O₂S₂), Zn(N₂S₂) or Zn(S₄) type.

The ligands in organic zinc (II) complexes according to the invention are preferably pyridine ring-containing ligands, pyrone ring-containing ligands, thiazolidine ring-containing ligands, thiophene ring-containing ligands,ligandscomprising amino acids, ligands comprising 2-aminothiophenol or ligands comprising dithiocarbamic acids.

The pyridine ring-containing ligands are preferably ligands having a pyridine ring and a carboxyl group or its derivative such as pyridine carboxylic acids, pyridylacetic acids, nicotinamides, picolinamides and the like, as well as ligands comprising 2-substituted-pyridine-N-oxides or the derivatives.

The pyrone ring-containing ligands are preferably ligands comprising 4-pyrone derivatives, more preferably 2-methyl-3-substituted-4-pyrone derivatives.

The thiazolidine ring-containing ligands are preferably ligands comprising a thiazolidine ring and a carboxyl group or its derivative such as thiazolidinecarboxylic acids, thiazolidineacetic acids, thiazolidinecarboxylic acid amides and the like.

The thiophene ring-containing ligands are preferably ligands comprising a thiophene ring and a carboxyl group or its derivative such as thiophenecarboxylic acids, thiopheneacetic acids, thiophenecarboxylic acid amides and the like.

The ligands comprising amino acids may for example be one comprising a compound having an amino group and a carboxyl group, preferably α-aminocarboxylic acids or the derivatives.

The preferred ligands comprising 2-aminothiophenols may for example be ligands comprising 2-aminothiophenols or the side chain-substituted derivatives.

The preferred ligands comprising dithiocarbamic acids may for example be ligands comprising N,N-dialkyl-substituted dithiocarbamic acids, N,N-diaryl-substituted dithiocarbamic acids or pyrrolidinedithiocarbamic acids and the side chain-substituted derivatives.

The preferred organic zinc (II) complexes according to the invention may for example be zinc (II) complexes of Zn(N₂O₂), Zn(O₄), Zn(O₂S₂), Zn(N₂S₂) or Zn(S₄) type comprising ligands such as pyridine ring-containing ligands, pyrone ring-containing ligands, ligands comprising amino acids, thiazolidine ring-containing ligands, thiophene ring-containing ligands, ligands comprising 2-aminothiophenols or ligands comprising dithiocarbamic acids as described above.

The preferred ligands of organic zinc (II) complexes of the invention are further detailed below.

The preferred examples of pyridine ring-containing ligands of organic zinc (II) complexes of the invention may for example be pyridine ring-containing carboxylic acid derivatives represented by Formula (1): 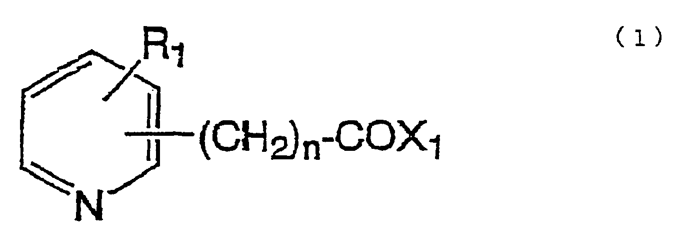 wherein R₁ is a hydrogen atom, an optionally substituted hydrocarbon group, a halogen atom, a hydroxyl group, an alkoxy group, an optionally substituted amino group or an optionally substituted heterocyclic group, and said R₁ may occur twice or more independently of one another on the pyridine ring, X₁ is a lower alkoxy group, an amino group optionally substituted by a lower alkyl group or a hydroxyl group, and n is an integer of 0 to 5.

A substituent R₁ in Formula (1) shown above is not limited particularly to those described above as long as insulin-like effects or hypoglycemic effects according to the invention is not affected adversely, and it may be bound in any position on the pyridine ring, and two or more substituents for example as R₁ groups may be bound on the pyridine ring.

A group -COX₁ denotes a carboxyl group or the derivatives, to which it is not limited as long as insulin-like effects or hypoglycemic effects according to the invention is not affected adversely. A group -(CH₂)ₙ-COX₁ may be bound in any position on the pyridine ring.

While n denotes an integer of 0 to 5 to which it is not limited as long as it enables a coordination with the center metal zinc and does not affect insulin-like effects or hypoglycemic effects of the invention adversely, it is preferably an integer of 0 to 5, more preferably 0 to 3, particularly 0 to 1.

Other preferred examples of pyridine ring-containing ligands of organic zinc (II) complexes of the invention are pyridine-N-oxide derivatives represented by Formula (2): 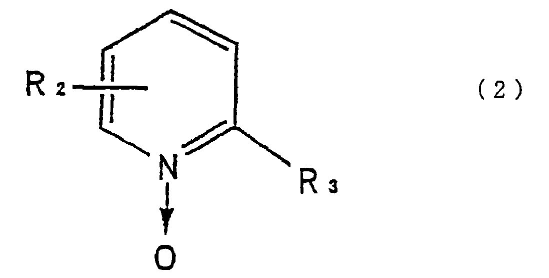 wherein R₂ is a hydrogen atom, an optionally substituted hydrocarbon group, a halogen atom, a hydroxyl group, an alkoxy group, an optionally substituted amino group or an optionally substituted heterocyclic group, and said R₂ may occur independently of one another twice or more on the pyridine ring and R₃ is a mercapto group, an optionally substituted amino group or a hydroxyl group.

A substituent R₂ in Formula (2) shown above is not limited particularly to those described above as long as insulin-like effects or hypoglycemic effects according to the invention is not affected adversely, and it may be bound in any of the 3-, 4-, 5- and 6- positions on the pyridine ring, and two or more substituents for example as R₂ groups may be bound on the pyridine ring.

While a group R₃ is not limited to a mercapto group, an optionally substituted amino group or a hydroxyl group as long as it can be bound to the center metal zinc via a coordinate bond or a covalent bond, it is preferably a mercapto group, an optionally substituted amino group or a hydroxyl group, more preferably a mercapto group or a hydroxyl group.

The preferred example of pyrone ring-containing ligands of organic zinc (II) complexes of the invention may for example be 4-pyrone derivatives, more preferably 2-substituted or unsubstituted methyl-3-substituted-4-pyrones or the derivatives. The preferred 4-pyrone derivatives may for example be 4-pyrone derivatives represented by Formula (3): 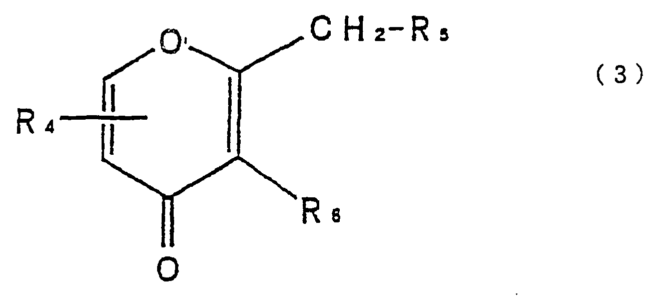 wherein each of R₄ and R₅ is independently of each other a hydrogen atom, an optionally substituted hydrocarbon group, a halogen atom, a hydroxyl group, an alkoxy group, an optionally substituted amino group or an optionally substituted heterocyclic group, and said R₄ may occur independently of one another twice or more on the pyrone ring, R₆ is a hydrogen atom, a mercapto group, an optionally substituted amino group or a hydroxyl group.

A substituent R₄ in Formula (3) shown above is not limited particularly to those described above as long as insulin-like effects or hypoglycemic effects according to the invention is not affected adversely, and it may be bound in any of the 5-and 6- positions on the pyrone ring, and two substituents for example as R₄ groups may be bound in both of the 5- and 6- positions on the pyrone ring.

A group R₅ is a substituent on the methyl group in the 2-position and not limited particularly to those described above as long as insulin-like effects or hypoglycemic effects according to the invention is not affected adversely, and the methyl group in the 2-position may be substituted by 2 or 3 substituent.

While a group R₆ is not limited to a hydrogen atom, a mercapto group, an optionally substituted amino group or a hydroxyl group as long as it can be bound to the center metal zinc via a coordinate bond or a covalent bond, it is preferably a hydrogen atom, a mercapto group, an optionally substituted amino group or a hydroxyl group, more preferably a hydroxyl group.

The ligands comprising amino acids of organic zinc (II) complexes of the invention may for example be α-amino acids or the derivatives, and more preferred examples are α-amino acids represented by Formula (4): 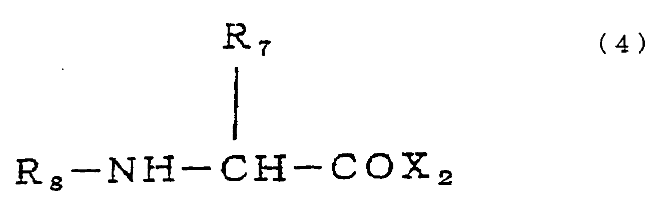 wherein R₇ is a hydrogen atom, an aralkyl group which is optionally substituted by a hydroxyl group or a lower alkoxy group, or a lower alkyl group which is optionally substituted by a substituted or unsubstituted heterocyclic group, hydroxyl group, a lower alkoxy group, guanidino group, amino group, carboxyl group, carbamoyl group, thiol group or a lower alkylthio group, or R₇ and R₈ are taken together to form a lower alkylene group; R₈ is same or different and denotes a hydrogen atom, a lower alkyl group or a lower alkylcarbonyl group; X₂ is a lower alkoxyl group, an amino group optionally substituted by a lower alkyl group or a hydroxyl group or a derivative.

A group R₇ is a substituent on an α carbon atom in an α-amino acid and not limited particularly to those described above as long as insulin-like effects or hypoglycemic effects according to the invention is not affected adversely.

A group -COX₂ denotes a carboxyl group or its derivative, to which it is not limited as long as an insulin-like effect or a hypoglycemic effect according to the invention is not affected adversely.

R₈ is a substituent on an amino group, and may preferably be, but not limited to, a free substituent, and may be a lower alkyl group or an acyl group such as a lower alkylcarbonyl group, a substituted amino group which is substituted by any of various amino protecting group employed in a peptide chemistry, and an amino group which formed a salt or a combination.

When R₇ and R₈ described above are taken together to form a lower alkylene group, the adjacent carbon and nitrogen atoms form a ring. For example when the lower alkylene group is a propylene group, it is taken together with the adjacent carbon and nitrogen atoms to form a nitrogen-containing 5-membered ring, whereby forming as an entire structure a proline which is a naturally occurring α-amino acid. Thus, α-amino acids according to the invention include amino acids containing rings such as proline.

The ligands comprising 2-aminothiophenols of organic zinc (II) complexes of the invention may for example be 2-aminothiophenol or the derivatives, and more preferred examples are 2-aminothiophenols represented by Formula (5): 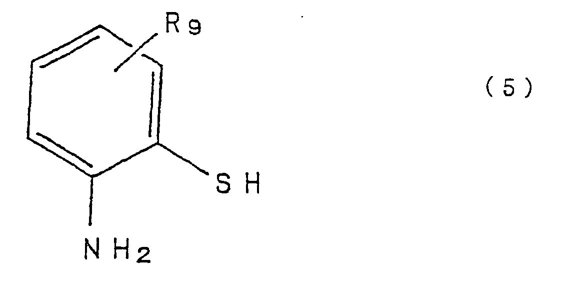 wherein R₉ is a hydrogen atom, an optionally substituted hydrocarbon group, a halogen atom, a hydroxyl group, an alkoxy group, an optionally substituted amino group or an optionally substituted heterocyclic group, and said R₉ may occur independently of one another twice or more on the benzene ring or the derivatives.

A substituent R₉ in Formula (5) shown above is not limited particularly to those described above as long as insulin-like effects or hypoglycemic effects according to the invention is not affected adversely, and it may be bound in any position on the benzene ring, and two or more substituents for example as R₉ groups may be bound on the benzene ring.

The ligands comprising dithiocarbamic acids of organic zinc (II) complexes of the invention may for example be dithiocarbamic acids or the derivatives, and more preferred examples are a dithiocarbamic acids represented by Formula (6): 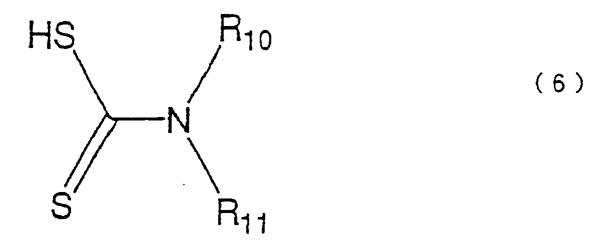 wherein each of R₁₀ and R₁₁ is a hydrogen atom or an optionally substituted hydrocarbon group; or R₁₀ and R₁₁ may be taken together to form an optionally substituted alkylene group or the derivatives.

The more preferred examples of dithiocarbamic acids or the derivatives represented by Formula (6) shown above is pyrrolidinedithiocarbamic acids represented by Formula (7): 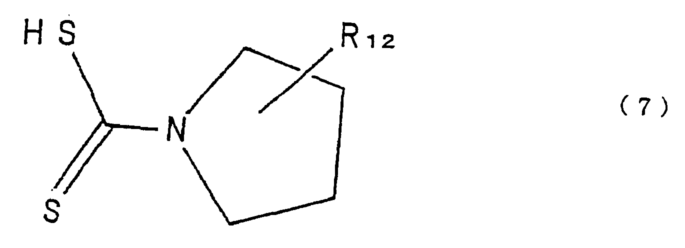 wherein R₁₂ is a hydrogen atom, an optionally substituted hydrocarbon group, a halogen atom, a hydroxyl group, an alkoxy group, an optionally substituted amino group or an optionally substituted heterocyclic group, and said R₁₂ may occur twice or more independently of one another on the pyrrolidine ring or the derivatives.

A substituent R₁₂ in Formula (7) shown above is not limited particularly to those described above as long as insulin-like effects or hypoglycemic effects according to the invention is not affected adversely, and it may be bound in any position on the pyrrolidine ring, and two or more substituents for example as R₁₂ groups may be bound on the pyrrolidine ring.

The preferred examples of thiazolidine ring-containing ligands of organic zinc (II) complexes of the invention is thiazolidine ring-containing carboxylic acid derivatives represented by Formula (8): 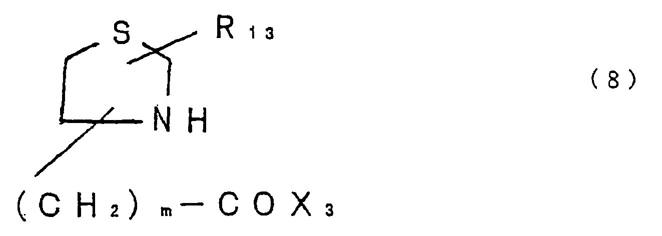 wherein R₁₃ is a hydrogen atom, an optionally substituted hydrocarbon group, a halogen atom, a hydroxyl group, an alkoxy group, an optionally substituted amino group or an optionally substituted heteroxyclic group, and said R₁₃ may occur twice or more independently of one another on the thiazolidine ring, X₃ is a lower alkoxy group, an amino group optionally substituted by a lower alkyl group or a hydroxyl group, and m is an integer of 0 to 5.

A substituent R₁₃ in Formula (8) shown above is not limited particularly to those described above as long as insulin-like effects or hypoglycemic effects according to the invention is not affected adversely, and it may be bound in any of the 2-, 4- and 5- positions on the thiazolidine ring, and two substituents for example as R₁₃ groups may be bound independently of each other in any two positions of 2-, 4- and 5-positions on the thiazolidine ring.

A group -COX₃ denotes a carboxyl group or its derivative, to which it is not limited as long as insulin-like effects or hypoglycemic effects according to the invention is not affected adversely. A group -(CH₂)ₘ-COX₃ may be bound in any of the 2-, 4- and 5- positions on the thiazolidine ring.

While m denotes an integer of 0 to 5 to which it is not limited as long as it enables a coordination with the center metal zinc and does not affect an insulin-like effect or a hypoglycemic effect of the invention adversely, it is preferably an integer of 0 to 5, more preferably 0 to 3, particularly 0 to 1.

The preferred examples of a thiophene ring-containing ligands of organic zinc (II) complexes of the invention are a thiophene ring-containing carboxylic acid derivatives represented by Formula (9): 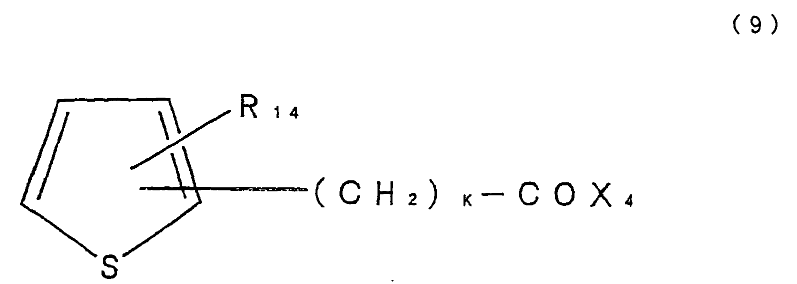 wherein R₁₄ is a hydrogen atom, an optionally substituted hydrocarbon group, a halogen atom, a hydroxyl group, an alkoxy group, an optionally substituted amino group or an optionally substituted heteroxyclic group, and said R₁₄ may occur twice or more independently of one another on the thiophene ring, X₄ is a lower alkoxy group, an amino group optionally substituted by a lower alkyl group or a hydroxyl group, and k is an integer of 0 to 5.

A substituent R₁₄ in Formula (9) shown above is not limited particularly to those described above as long as an insulin-like effect or a hypoglycemic effect according to the invention is not affected adversely, and it may be bound in any position on the thiophene ring, and two or more substituents for example as R₁₄ groups may be bound on the thiophene ring.

A group -COX₄ denotes a carboxyl group or its derivative, to which it is not limited as long as an insulin-like effect or a hypoglycemic effect according to the invention is not affected adversely. A group -(CH₂)ₖ-COX₄ may be bound in any position on the thiophene ring.

While k denotes an integer of 0 to 5 to which it is not limited as long as it enables a coordination with the center metal zinc and does not affect insulin-like effects or hypoglycemic effects of the invention adversely, it is preferably an integer of 0 to 5, more preferably 0 to 3, particularly 0 to 1.

The substituents R₁, R₂, R₄, R₉, R₁₂, R₁₃, R₁₄ on the pyridine, pyrone, benzene, pyrrolidine, thiazolidine and thiophene rings and a substituent R₅ on the methyl group in the 2-position of the pyrone ring may be any of various organic residues as long as they do not affect an insulin-like effect or a hypoglycemic effect of the invention adversely, and those employed preferably be optionally substituted hydrocarbon groups, halogen atoms, hydroxyl group, alkoxy groups, optionally substituted amino groups, optionally substituted heterocyclic groups and the like.

The hydrocarbon groups may for example be lower alkyl groups, lower alkenyl groups, lower alkynyl groups, cycloalkyl groups, cycloalkenyl groups, aryl groups and the like.

The "lower alkyl group" employed in the invention may for example be a straight or branched alkyl group having 1 to 15, preferably 1 to 10, more preferably 1 to 6, particularly 1 to 4 carbon atoms, typically including methyl, ethyl, propyl, isopropyl, butyl, isobutyl, s-butyl, t-propyl, pentyl, hexyl groups and the like. The "lower alkenyl group" may for example be a straight or branched alkenyl group having 2 to 15, preferably 2 to 10, more preferably 2 to 6 carbon atoms, typically including vinyl, allyl, butenyl, pentenyl groups and the like. The "lower alkynyl group" may for example be a straight or branched alkynyl group having 2 to 15, preferably 2 to 10, more preferably 2 to 6 carbon atoms, typically including ethynyl, propynyl, butynyl, pentynyl groups and the like.

The "cycloalkyl group" may for example be a monocyclic, polycyclic or fused cycloalkyl group having 3 to 30, preferably 5 to 20, more preferably 6 to 10 carbon atoms, and the "cycloalkenyl group" may for example be a cycloalkyl group mentioned above further containing one or more unsaturated groups such as a double bond.

The "aryl group" may for example be a monocyclic, polycyclic or fused aromatic hydrocarbon group having 6 to 30, preferably 6 to 20, more preferably 6 to 10 carbon atoms.

The "lower alkylene group" may for example be a straight or branched alkylene group having 1 to 15, preferably 1 to 10, more preferably 1 to 6, particularly 1 to 4 carbon atoms. When such a lower alkylene group is taken together with an adjacent atom to form a ring, one or more of the carbon atoms of the lower alkylene group may be replaced with an oxygen, nitrogen or sulfur atom.

The "hydrocarbon group" described above may further contain a substituent. Such a substituent may for example be a lower alkoxy group derived from a lower alkyl group described above, a hydroxy group, an amino group, a halogen atom, a heterocyclic group, a nitro group and the like.

The "halogen group" may for example be chlorine, bromine, iodine and the like.

The "alkoxy group" may for example be a lower alkoxy group derived from a lower alkyl group described above. The substituent on an "optionally substituted amino group" may for example be a hydrocarbon group described above and an acyl group derived from such a hydrocarbon group.

The "heterocyclic group" may for example be a saturated or unsaturated, monocyclic, polycyclic or fused ring having in its ring at least one nitrogen atom, oxygen atom or sulfur atom in which each ring is 5- to 20-membered, preferably 5-to 10-membered, more preferably 5- to 7-membered and which may be fused with a hydrocarbon group such as a cycloalkyl, cycloalkenyl or aryl group.

The heterocyclic group described above may further have a substituent, which may for example be a lower alkoxy group derived from the lower alkyl group described above, a hydroxy group, an amino group, a halogen atom, a heterocyclic group, a nitro group and the like.

The organic zinc (II) complexes of the invention can be produced by known methods (for example, US Patent No.5,219,847) or the methods according to such known methods. For example, the solution of an intended ligand is combined with the solution of a zinc salt to form an organic zinc (II) complex, which is then isolated as a product. While a preferred solvent is usually water, organic solvents or solvent mixtures may also be employed. The solution of a zinc salt is preferably the aqueous solution of an inorganic zinc such as zinc sulfate, zinc nitrate, zinc chloride and the like. It may sometimes be preferred to adjust the pH of the reaction mixture. The pH modifier may be the basic aqueous solution of sodium hydroxide, lithium hydroxide, barium hydroxide and the like.

More typically, examples described below can be followed.

The organic zinc (II) complexes of the invention have insulin-like effects or hypoglycemic effects as evidents also from experiments described below, and are useful as prophylactic or therapeutic agents against diabetes or hypertension.

Accordingly, the invention provides pharmaceutical compositions comprising organic zinc (II) complexes of the invention described above and pharmaceutically acceptable carrier.

In order to obtain the formulation, the organic zinc (II) complexes according to the invention are employed as active ingredients to form pharmaceutical formulations together with pharmaceutically acceptable carriers such as organic or inorganic, solid or liquid excipients suitable for oral, parenteral or topical administrations. Such pharmaceutical formulations may for example be capsule, tablet, syrup, granule, inhalant, suppository, liquid, lotion, suspension, emulsion, ointment, gel and the like. If necessary, any of the formulations described above may contain auxiliary agents, stabilizers, humectants or emulsifiers, buffering agents or other customary additives.

The invention also provides oral formulations comprising organic zinc (II) complexes of the invention. The inventive oral formulations are oral formulations comprising organic zinc (II) complexes having insulin-like effects or hypoglycemic effects. Accordingly, the invention provides a prophylactic or therapeutic agent against diabetes which can orally be administered. Those also provided are the use of organic zinc (II) complexes for producing prophylactic or therapeutic agents against diabetes which can orally be administered and methods for preventing or treating diabetes by means of oral administrations of therapeutically effective amounts of prophylactic or therapeutic agents against diabetes which can orally be administered.

While the therapeutically effective amount of organic zinc (II) complexes of the invention may vary depending on the ages and the condition of the patients to be treated, the dosage which give about 0.1 mg/patient or about 1000 mg/patient as mean single doses of the organic zinc (II) complexes of the invention may be given once to several times a day.

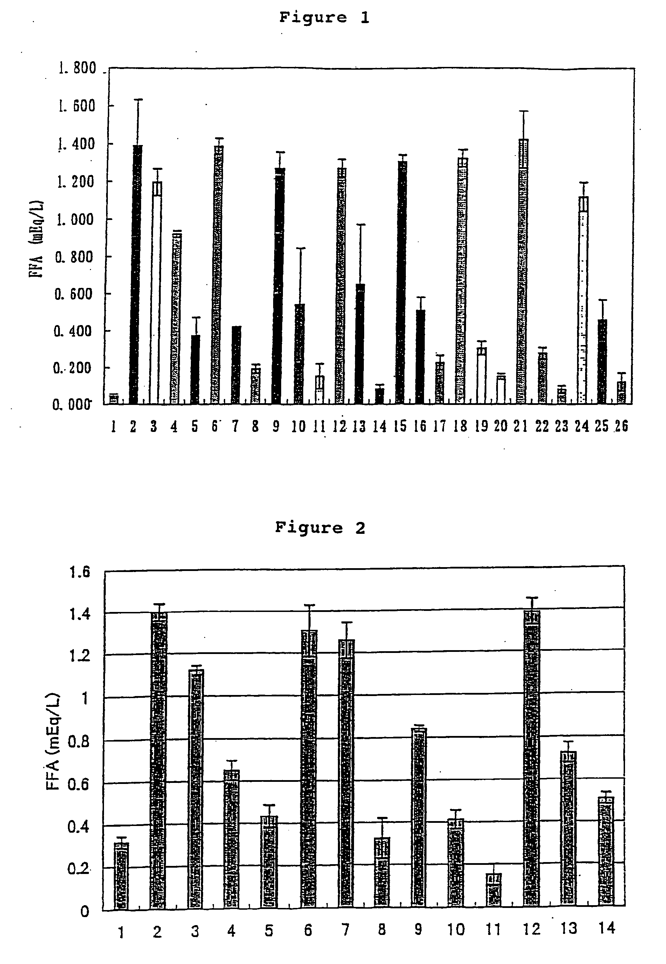

The results of the study on the inhibitory effect of organic zinc (II) complexes of the invention on the release of a fatty acid in rat fat cells are shown in Figure 1 and Figure 2.

Each of Figures 1 and 2 shows the free fatty acid inhibiting effect observed when an organic zinc (II) complex of the invention was added to an epinephrine-stimulated rat adipocyte.

In Figure 1, the results are designated by 1 for a blank, 2 for a control, 3 to 5 for positive controls using oxovanadium sulfate (VOSO₄) and 6 to 26 for inventive compounds.

The results are designated by 6 to 8 for zinc (II) picolic acid complex [Zn(PA)₂(H₂O)₂], 9 to 11 for zinc (II) 6-methyl-picolic acid complex [Zn(MPA)₂(H₂O)], 12 to 14 for zinc (II) pyridine-2-acetic acid complex [Zn(PAA)₂(H₂O)₂], 15 to 17 for zinc (II) glycine complex [Zn(Gly)₂(H₂O)], 18 to 20 for zinc (II) threonine complex [Zn(Thr)₂(H₂O)₂], 21 to 23 for zinc (II) maltol complex · 2.6 hydrate Zn(maltol)₂/2.6H₂O and 24 to 26 for zinc (II) 2-mercaptopyridine-N-oxide complex [(Zn(MPNO)₂)₂].

Each compound of 3 to 23 was employed at 10⁻⁴M, 5 x 10⁻⁴M, 10⁻³M, and 24 to 26 at 5 x 10⁻⁵M, 2.5 x 10⁻⁴M, 5 x 10⁻⁴M.

In Figure 2, the results are designated by 1 for a blank, 2 for a control, 3 to 5 for positive controls using oxovanadium sulfate (VOSO₄) and 6 to 14 for inventive compounds.

The results are designated by 6 to 8 for zinc (II) 2-aminothiophenol complex [Zn(ABT)₂], 9 to 11 for zinc (II) pyrrolidine-N-dithiocarbamate complex [Zn(PCD)₂] and 12 to 14 for zinc (II) cysteine methyl ester complex [Zn(Cys-OMe)₂].

Each compound of 3 to 5 and 12 to 14 was employed at 10⁻⁴M, 5 x 10⁻⁴M, 10⁻³M, and 6 to 8 and 9 to 11 at 10⁻⁵M, 5 x 10⁻⁵M, 10⁻⁴M.

In Figure 1 and Figure 2, the "blank" designated by 1 indicates the free fatty acid (FAA) level due to the spontaneous release by cells, while the "control" designated by 2 indicates the free level due to the stimulation by epinephrine. The "VOSO₄" in 3 to 5 in Figure 1 and Figure 2 means oxovanadium sulfate as a comparative example.

The IC₅₀(mM) values as the test substance concentrations capable of inhibiting the release of the fatty acid by 50 % calculated for inventive zinc (II) complexes based on the results described above and the results obtained similarly for other inventive zinc (II) complexes than those described above are shown in Table 1. The relative values on the bas is of the IC₅₀(mM) of "VOSO₄" being regarded as 1.00 mM are indicated in Table 1. 6-Methyl-picolinic acid complex [Zn(MPA)₂(H₂O)] and zinc (II) pyridine-2-acetic acid complex [Zn(PAA)₂(H₂O)₂] were determined in DMSO (dimethylsulfoxide) and zinc (II) glycine complex [Zn(Gly)₂(H₂O)] was determined in an aqueous solution, while other test substances were determined in physiological saline.

**Table 1**

| Complex | IC₅₀(mM) | | Remarks |
|---|---|---|---|
| | Glucose + | Glucose - | |
| trans-(Zn(PA)₂(H₂O)₂] | 0.50 | 0.57 | PA=Picolic acid |
| Zn(6MPA)₂(H₂O) | 0.39 | | 6MPA=6-Methylpicolic acid |
| Zn(3MPA)₂ | | 0.42 | 3MPA=3-methylpicolic acid |
| Zn(6BPA)₂ | | 0.45 | 6BPA=6-Bromopicolic acid |
| Zn(PAA)₂/2.4H₂O | 0.39 | | PAA=Pyridyl-2-acetic acid |
| Zn(Nic)₂/4H₂O | 0.82 | | Nic=Nicotinic acid |
| Zn(Gly)₂/1.0H₂O | 0.63 | 0.84 | Gly=Glycine |
| cis-[Zn(D-Thr)₂(H₂O)₂] | | 1.15 | Thr=Threonine |
| cis-[Zn(Thr)₂(H₂O)₂] | 0.54 | 1.30 | Glu=Glutamine |
| Zn(Gln)₂ | 0.84 | | 4Tia=Thiazolidine-4-carboxylic acid |
| Zn(D-4Tia)₂ | | 1.28 | Koj=Kojic acid |
| Zn(L-4Tia)₂ | | 1.20 | Maltol=3-Hydroxy-2-methyl-4-pyrone |
| Zn(Koj)₂ | | 0.91 | HPNO=2-Hydroxypyridine-N-oxide |
| Zn(maltol)₂/2.6H₂O | 0.57 | | |
| Zn(HPNO)₂ | | 0.41 | ABT=2-Aminothiophenol |
| Zn(ABT)₂ | | 0.27 | Cys-OMe=Cysteine methyl ester |
| Zn(Cys-OMe)₂ | | 1.53 | |
| Zn(TC)₂ | 0.54 | 1.43 | TC=Thiopehene-2-carboxylic acid |
| Zn(MPNO)₂/0.3H₂O | 0.011 | 0.013 | MPNO=2-Mercaptopyridine N-Oxide |
| Zn(PCD)₂ | | 0.049 | |
| | | | PCD=Pyrrolidine-N-dithiocarbamate |
| VOSO₄/5.4 or 2.3H₂O | 1.00 | 1.00 | VOSO₄=Oxovanadium sulfate |

As evident from these results, an organic zinc (II) complex of the invention exhibited a significant inhibitory effect on the release of a fatty acid from a rat adipocyte when compared with VOSO₄ and thus was proven to be excellent as a prophylactic or therapeutic agent against a diabetes or a hypertension.

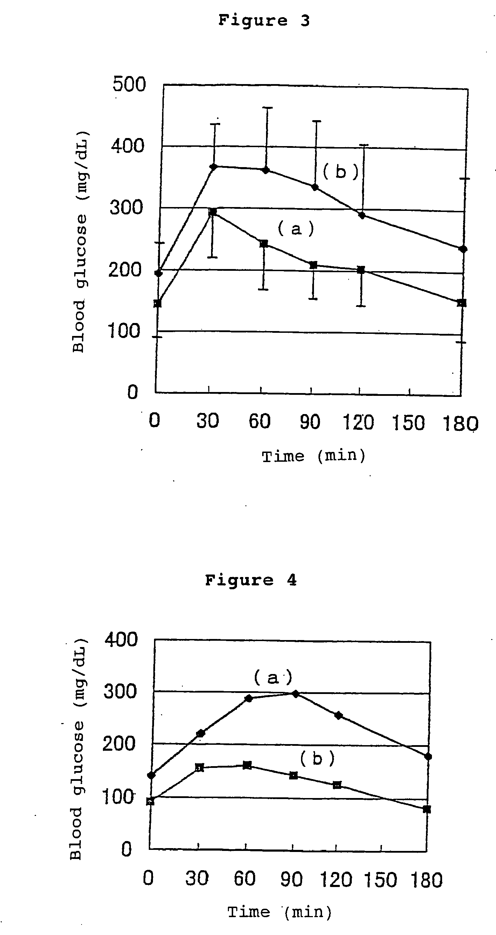

Subsequently, KK-A^{y} mice as type II diabetes model animals and humans were subjected to the glucose tolerance tests to obtain blood glucose curves, which are shown in Figure 3 and Figure 4.

In Figure 3, (a) denotes a blood glucose curve obtained in a glucose tolerance test in mice each of which received 4 mg/kg of Zn(PA)₂(H₂O)₂ as an inventive zinc (II) complex once a day for 3 days and then fasted for 14 hours, and (b) denotes one in a control group in which the mice were not treated with the complex. In Figure 4, (a) denotes a blood glucose curve obtained in a glucose tolerance test in diabetes patients, while (b) denotes one in normal healthy volunteers.

Figure 4 shows the results of a glucose tolerance test employed in human diabetes tests [A.HAGURA, "NIPPON RINSHO (extra edition), Diabetes 3", in-all vol.728, p419 "KK NIPPONRINSHOSHA"], and reveals that a volunteer whose glucose metabolism was normal exhibited a slight increase in the blood glucose after an oral administration of glucose followed by a rapid decrease and then recovered the level before the administration after 180 minutes. On the other hand, a diabetes patient whose glucose metabolism was abnormal exhibited an increase in the blood glucose to the level approximately twice that in the normal healthy volunteers which was kept for a while and did not recover the level before the glucose administration even after 180 minutes.

On the contrary, a glucose tolerance test in KK-A^{y} mice revealed, as evident from Figure 3, that each mouse receiving 4 mg/kg of Zn(PA)₂(H₂O)₂ as an inventive zinc (II) complex once a day for 3 days and then fasting for 14 hours exhibited, after administering glucose, a reduced peak level of the blood glucose followed by a rapid decrease, as compared with the mice not receiving the complex and then recovered its almost normal blood glucose level after 180 minutes.

Based on the results described above, the type II diabetes was ameliorated in the mice receiving the inventive zinc (II) complex.

### Examples

The invention is further detailed in the following examples, which are not intended to restrict the invention.

### Example 1

### Bis(picolinate) zinc (II) complex [Zn(PA)₂(H₂O)₂] production

According to the method by Lumme et al (P.Lumme et al., Acta Chemica Scandinavica, 23, 3011-22 (1969)), picolic acid (10 mM) was dissolved in 10 ml of an aqueous solution of lithium hydroxide (10 mM). To this solution, an aqueous solution of zinc (II) sulfate (5 mM) was added dropwise in potions with stirring. After several days, the precipitation formed was recovered by a filtration, washed thoroughly with water, dried to obtain 3.26 g of the desired product as a white substance (yield 90 %).

The resultant trans-bis(picolinate) zinc (II) complex [Zn(PA)₂(H₂O)₂] had the structure shown below.

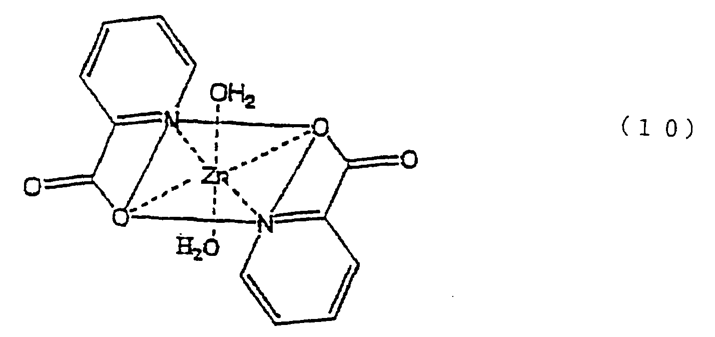

### Example 2

According to the method in Example 1, zinc (II) 6-methyl-picolinic acid complex [Zn(MPA)₂(H₂O)] was produced.

The resultant bis (6-methyl-picolinate) zinc (II) complex [Zn(MPA)₂(H₂O)] had the structure shown below.

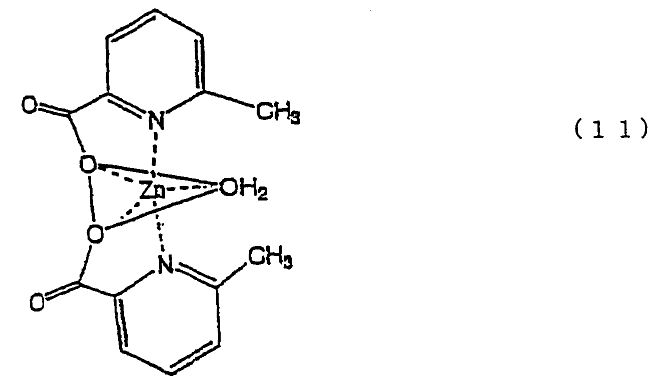

### Example 3

According to the method by Faure et al (P.R.Faure et al., Acta Cryst., B28, 811-5(1972)), bis(pyridine-2-acetate) zinc (II) complex [Zn(PAA)₂(H₂O)₂] was produced.

The resultant trans-bis(pyridine-2-acetate) zinc (II) complex [Zn(PAA)₂(H₂O)₂] had the structure shown below.

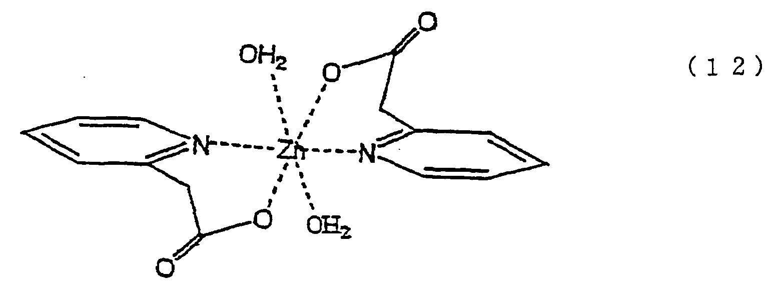

### Example 4

According to the method by Hamalainen (R.Hamalainen, Finn.Chem.Lett., 1977, 113), bis(threonine) zinc (II) complex [Zn(Thr)₂(H₂O)₂] was produced.

The resultant cis-bis(threonine) zinc (II) complex [Zn(Thr)₂(H₂O)₂] had the structure shown below.

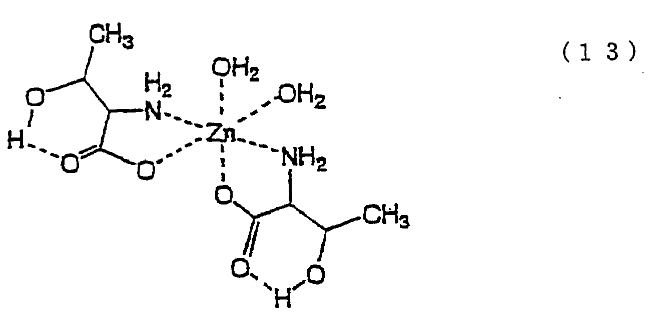

### Example 5

According to the method by Newman et al (J.M.Newman et al., Acta Cryst., C46, 44-8 (1990)), bis(glycine) zinc (II) complex [Zn(Gly)₂(H₂O)] was produced.

The resultant trans-bis(glycine) zinc (II) complex [Zn(Gly)₂(H₂O)] had the structure shown below.

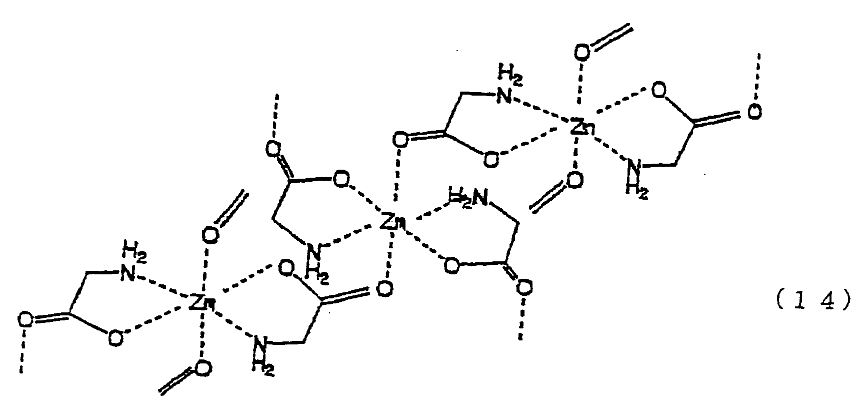

### Example 6

### Production of bis(3-hydroxy-2-methyl-4-pyronate) zinc (II) complex [Zn(maltol)₂/2.6H₂O]

Maltol (3-hydroxy-2-methyl-4-pyrone) (10 mM) was dissolved in 10 ml of an aqueous solution of lithium hydroxide (10 mM). To this solution, an aqueous solution of zinc (II) sulfate heptahydrate (5 mM) was added dropwise in potions with stirring. The precipitation formed was recovered by a filtration, washed thoroughly with water, dried to obtain 0.588 g of the desired product as a white substance (yield 32.5 %). Molecular formula: Zn(C₆H₅O₃)₂/2.6H₂O Molecular weight: 361.8

| Elemental analysis: | | | |
|---|---|---|---|
| Calc. (%) | C:39.77; | H:4.23; | N:0.00 |
| Found (%) | C:39.66; | H:4.14; | N:0.02 |

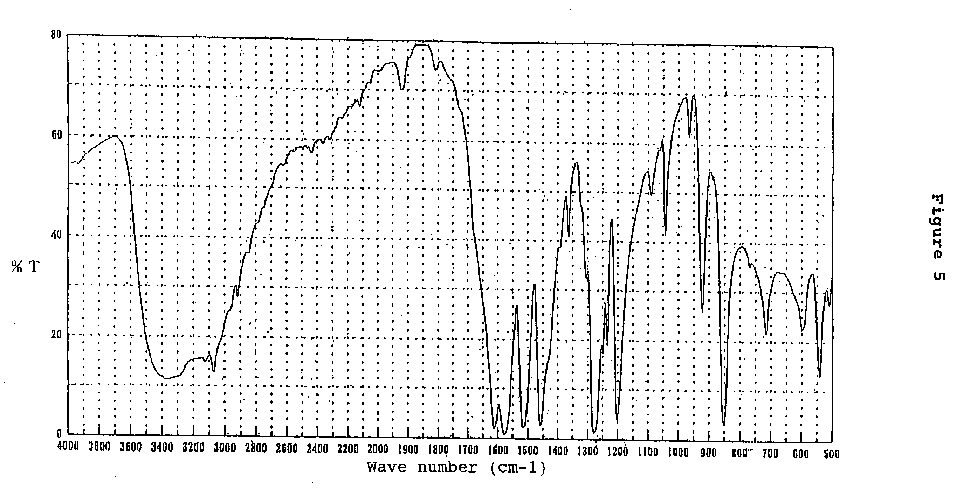

IR: Full chart shown in Figure 5

### Example 7

### Production of bis(2-mercaptopyridine-N-oxide) zinc (II) complex [(Zn(MPNO)₂)₂]

2-Mercaptopyridine-N-oxide (10 mM) was dissolved in 10 ml of an aqueous solution of sodium hydroxide (10 mM). After 30 minutes, the solution was adjusted at pH 8 with 1 M HCl. After further 30 minutes, an aqueous solution of zinc (II) sulfate heptahydrate (5 mM) was added portionwise with stirring. The precipitation formed was recovered by a filtration, washed thoroughly with water, dried to obtain 0.714 g of the desired product as a white substance (yield 44.2 %).

The resultant bis(2-mercaptopyridine-N-oxide) zinc (II) dinuclear complex had the structure shown below (B.L.Barnett et al., Inorg.Chem., 16, 1834-1838 (1977)).

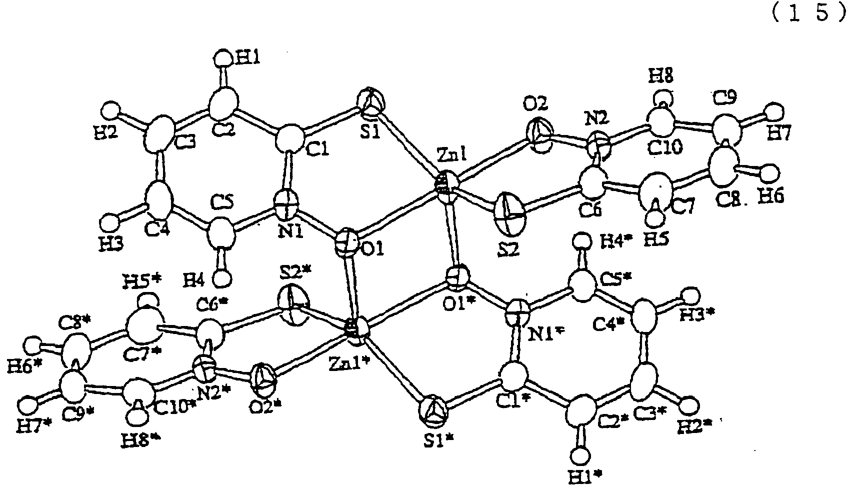

### Example 8

### Production of bis(2-aminothiophenol) zinc (II) complex [Zn(ABT)₂/0.5H₂O]

A solution of 250 mg (2 mM) of 2-aminobenzenethiol (ABT) in methanol was combined with an aqueous solution of 85 mg (2 mM) of lithium hydroxide, and the solution was combined after several hours with an aqueous solution of 288 mg (1 mM) of zinc (II) sulfate heptahydrate. After allowing to stand with stirring overnight, insolubles were recovered by a filtration, washed several times with a pure water, dried under reduced pressure to obtain the desired substance at 78 % yield.

### Example 9

### Production of bis(pyrrolidine-N-dithiocarbamate) zinc (II) complex [Zn(PCD)₂/0.2H₂O]

492 mg (3 mM) of ammonium pyrrolidine-N-carbodithioate (APCD) was dissolved in 5 ml of a 0.8 M aqueous solution of sodium hydroxide, and the solution was adjusted at pH8 with 1M hydrochloric acid. The solution was combined further with an aqueous solution of 431 mg (1.5 mM) of zinc (II) sulfate heptahydrate, whereupon the pH became approximately 6. After several hours, the white precipitate formed was recovered by a filtration, washed several times with a pure water, dried under reduced pressure to obtain the intended substance at 96 % yield.

### Example 10

### Production of bis(L-cysteine methyl ester) zinc (II) complex [Zn(Cys-OMe)₂]

An aqueous solution of 514 mg (3 mM) of L-cysteine methyl ester hydrochloride [(Cys-OMe) · HCl] was combined with an aqueous solution of 945 mg (3 mM) of barium hydroxide octahydrate, and the solution was combined after several hours with an aqueous solution of 431 mg (1.5 mM) of zinc (II) sulfate heptahydrate. After allowing to stand with stirring overnight, insolubles (precipitates) were separated by a filtration, and the filtrate was concentrated to an about half volume, and the precipitate formed was recovered by a filtration, washed several times with methanol, dried under reduced pressure to obtain the intended substance at 42 % yield.

### Pharmacological test example 1

A pharmacological test was conducted as described below in accordance with the method reported in Biol.Pharm.Bull., 18, 719-725 (1995).

### (1) Preparation of rat adipocyte

A male Wistar rat weighing 200 g was exsanguinated under an anesthesia with ether, and a fat cell mass was isolated from a fat tissue around an epididymis according to the method of Road Bell (J. Biol. Chem., 239, 375(1964)). The fat cell mass was cut into pieces using scissors, and digested for 1 hour at 37°C in a KRB buffer solution (5 mM Glucose, 120 mM NaCl, 1.27 mM CaCl₂, 1.2 mM MgSO₄, 4.75 mM KCl, 1.2 mM KH₂PO₄ and 24 mMNaHCO₂; pH=7.4) supplemented with 20 mg of bovine serum albumin (BSA) and 2 mg of collagenase per ml. The fat cells were sieved through a nylon mesh (250 µm) whereby being made free from any undigested tissue, washed three times with the buffer similar to that described above except for containing no collagenase, and adjusted at 2.5 x 10⁶ cells/ml.

### (2) Effect of zinc (II) complex on rat adipocyte

The adipocyte separated as described above contained in a silicone-treated vial (2.5 x 10⁶ cells/ml) were preincubated for 0.5 hours at 37°C in 1 ml of a KRB buffer solution supplemented with 20 mg BSA/ml in the presence of VOSO₄ and each inventive organic Zn (II) complex at a varying concentration (10⁻⁴, 5x10⁻⁴, 10⁻³). Then the reaction mixture was combined with 10⁻⁵ M epinephrine and the resultant solution was incubated for 3 hours at 37°C. The reaction was quenched by cooling with ice, and the mixture was centrifuged for 10 minutes at 3000 rpm. The extracellular solution was examined for its free fatty acid (FFA) level using an NEFA kit whereby calculating an IC₅₀ value.

The results are shown in Figure 1, Figure 2 and Table 1.

Based on the results described above, each organic zinc (II) complex of the invention was proven to be capable of inhibiting the release of the fatty acid from the rat adipocyte significantly when compared with VOSO₄, thus being excellent as a drug for treating diabetes.

### Pharmacological test example 2

### (1) Methods

8-Week old KK-A^{y} mice were treated intraperitoneally once a day with a 5 % acacia solution in a control group (n=6) or with Zn(PA)₂ (n=6), Zn(maltol)₂ (n=7) or Zn(Cys-OMe)₂ (n=6) dissolved in a 5 % acacia solution in the complex treatment groups.

The mice exhibiting blood glucose levels of 200 mg/d L or higher were treated at 4.5 mg Zn/kg and the mice of 150 mg/d L or higher but less than 200 mg/dL at 2.5 mg Zn/kg, while the mice of less than 150 mg/dL were not treated.
In the control group, 0.5 ml of the acacia solution was given.

The onset of a diabetes was ensured on the basis of a mean blood glucose level immediately before the treatment of 450 mg/dL or higher and a mean body weight of 35 g or higher.

The blood glucose was determined using a simplified blood sugar tester (GLUCOCARD, manufactured by KYOTO DAIICH KAGAKU).

### (2) Results and discussion

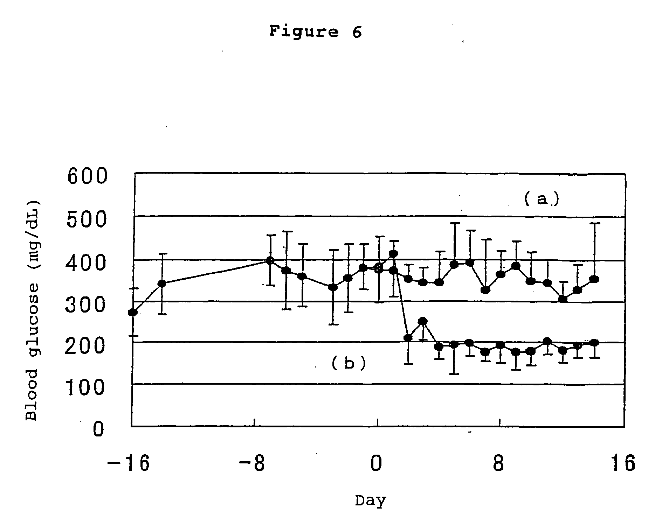

The blood glucose change after the treatment with Zn(PA)₂ for 15 days is shown in Figure 6.

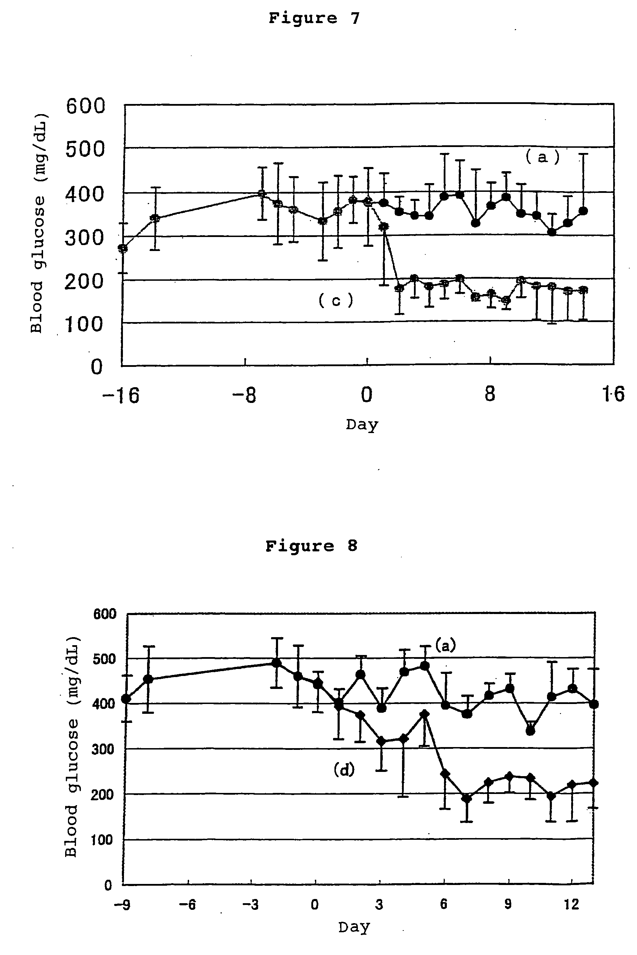

The blood glucose change after the treatment with Zn(maltol)₂ for 15 days is shown in Figure 7.

The blood glucose change after the treatment with Zn(Cys-OMe)₂ for 13 days is shown in Figure 8.

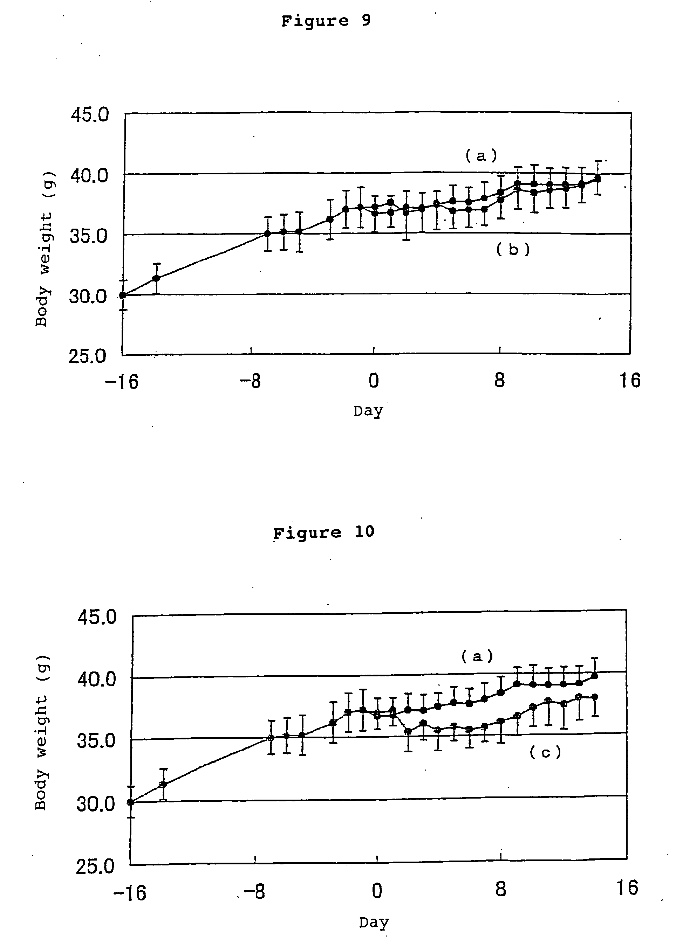

The body weight change after the treatment with Zn(PA)₂ for 15 days is shown in Figure 9.

The body weight change after the treatment with Zn(maltol)₂ for 15 days is shown in Figure 10.

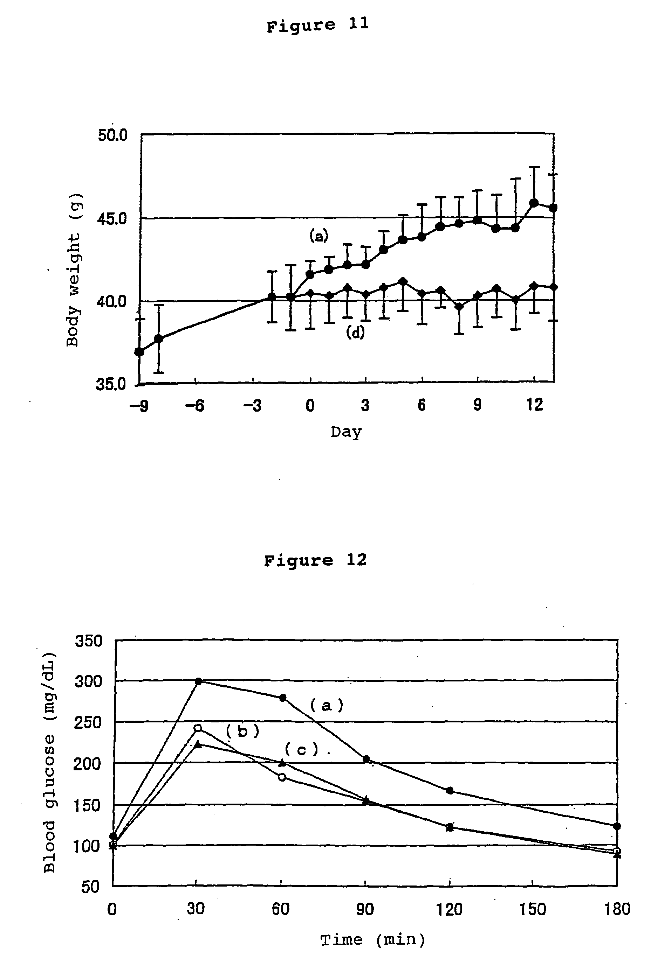

The body weight change after the treatment with Zn(Cys-OMe)₂ for 13 days is shown in Figure 11.

The results of a glucose tolerance test after a treatment with a complex for 15 days are shown in Figure 12. (glucose tolerance test: A mouse was fasted for 13 hours and then administered orally with glucose at 1 g/kg, and then the blood glucose levels were determined at certain time intervals.)

In Figures 6 to 12, the results are designated by (a) in the control group, by (b) in the group treated with the complex Zn(PA)₂, by (c) in the group treated with the complex Zn (maltol)₂, by (d) in the group treated with the complex Zn(Cys-OMe)₂.

Any of the groups treated with the three complexes was highly effective in normalizing the blood glucose level as compared with the control group (Figure 6 to Figure 8).

The weight loss, which is an index of side effects, was not observed in most of the animals treated with Zn(PA)₂ (Figure 9).

While the weight loss was observed in Zn(maltol)₂ group immediately after the treatment with the complex, the body weight was recovered in response to the reduction in the blood glucose and the reduction in the dose (Figure 10).

In Zn(Cys-OMe)₂ group, no increase or decrease in the body weight was observed (Figure 11). These findings were considered to be indicative of an amelioration of the diabetes reflected by the inhibition of the weight gain in obese KK-A^{y} mice as the type II diabetes model animals.

Also using Zn(PA)₂ complex and Zn(maltol)₂ complex, the glucose tolerance test was conducted after the administration of each complex for 15 days, and the results revealed an amelioration of the diabetes conditions when compared with the diabetes mice (control mice) (Figure 12).

### Industrial Applicability

The organic zinc (II) complexes of the invention are highly stable and have fat-soluble insulin-like effects and hypoglycemic effects. Accordingly, the organic zinc (II) complexes of the invention are useful as prophylactic or therapeutic agent against glucose tolerance impairment, diabetes (such as type II diabetes), insulin resistant syndrome (such as insulin receptor dysfunction), polycystic ovary syndrome, hyperlipidemia, atherosclerosis, cardiovascular disease (such as angina pectris, heart failure), hyperglycemia or hypertension, or angina pectris, hypertension, pulmonary hypertension, congestive heart failure, diabetic complication (such as diabetic gangrene, diabetic arthropathy, diabetic glomeruloscrelosis, diabetic dermopathy, diabetic neuropathy, diabetic cataract, diabetic retinophathy).

## Claims

1. The hypoglycemic agents comprising organic zinc (II) complexes.

2. The hypoglycemic agents according to Claim 1 wherein said hypoglycemic agents are a prophylactic or therapeutic agents against diabetes.

3. The hypoglycemic agents according to Claim 1 or 2 wherein said organic zinc (II) complexes have thiazolidine ring-containing ligands.

4. The hypoglycemic agents according to Claim 3 wherein said thiazolidine ring-containing ligands are thiazolidine ring-containing carboxylic acid derivatives represented by Formula (8): 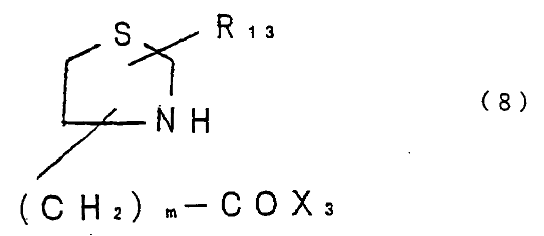 wherein R₁₃ is a hydrogen atom, an optionally substituted hydrocarbon group, a halogen atom, a hydroxyl group, an alkoxy group, an optionally substituted amino group or an optionally substituted heteroxyclic group, and said R₁₃ may occur twice or more independently of one another on the thiazolidine ring, X₃ is a lower alkoxy group, an amino group optionally substituted by a lower alkyl group or a hydroxyl group, and m is an integer of 0 to 5.

5. The hypoglycemic agents according to any one of Claims 1 to 4 wherein said organic zinc (II) complexes are zinc (II) complexes of Zn(N₂O₂), Zn(O₄), Zn(O₂S₂), Zn(N₂S₂) or Zn(S₄) type.

6. The oral formulations comprising organic zinc (II) complexes.

7. The oral formulations according to Claim 6 wherein said oral formulations comprise organic zinc (II) complexes having insulin-like effects.

8. The oral formulations according to Claim 6 or 7 wherein said oral formulations are hypoglycemic agents.

9. the oral formulations according to Claim 6 or 8 wherein said organic zinc (II) complexes are an organic zinc (II) complexes containing ligands according to any one of Claims 3 to 4.
